# EUROPEAN PATENT APPLICATION

(11) **EP 4 056 184 A1**
(43) Date of publication of application: **14.09.2022**
(21) Application number: 20884271.6
(22) Date of filing: 09.11.2020
(51) Int. Cl.: A61K 31/5377, A61K 31/52, C07D 473/16, C07D 473/18, C07D 473/32, A61P 35/02

(54) **USE OF MULTI-TARGET PROTEIN KINASE INHIBITOR**

(30) Priority: 09.11.2019 CN 201911090822
(71) Applicant: CSPC Zhongqi Pharmaceutical Technology (Shijiazhuang) Co., Ltd., High-Tech Development Zone Shijiazhuang Hebei 050035 (CN)
(72) Inventor: PENG, Yueying, Shijiazhuang, Hebei 050035 (CN); ZHAN, Guoning, Shijiazhuang, Hebei 050035 (CN); LI, Xiaojing, Shijiazhuang, Hebei 050035 (CN); HAO, Chunyan, Shijiazhuang, Hebei 050035 (CN); ZHAO, Xuemin, Shijiazhuang, Hebei 050035 (CN); HU, Wei, Shijiazhuang, Hebei 050035 (CN)
(74) Representative: Longland, Emma Louise
(86) International application number: PCT/CN2020/127449
(87) International publication number: WO 2021/089038

(57) **Abstract**

The present invention provides use of a multi-target protein kinase inhibitor compound A or a pharmaceutically acceptable salt thereof in the manufacture of a medicament for treating leukemia, and provides a method for treating acute myeloid leukemia, especially acute myeloid leukemia with FLT3 mutation, with compound A. In clinical trials, compound A has certain efficacies both on acute myeloid leukemia with FLT3-ITD mutation and/or FLT3-TKD mutation, and on DEK-CAN positive acute myeloid leukemia with FLT3-ITD mutation. Patients with relapsed and/or refractory acute myeloid leukemia who have failed treatment previously with Type II FLT3 inhibitors (e.g., sorafenib) can still clinically benefit from the treatment with compound A.

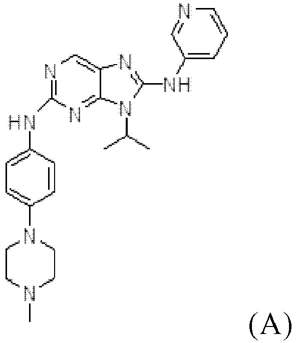

## Description

### Technical Field

The invention belongs to the field of medicine, and in particular, relates to use of a multi-target protein kinase inhibitor in the manufacture of a medicament for treating leukemia and a method for treating the above-mentioned disease.

### Background technology

Acute myeloid leukemia (AML) is a heterogeneous hematological malignancy characterized by the proliferation of myeloid blast cells in the bone marrow, peripheral blood, and/or other tissues. The main clinical manifestations are infection, hemorrhage, anemia and extramedullary tissue and organ infiltration. The disease progresses rapidly, and the natural disease course is only from a few weeks to several months. AML is the most common type of adult acute leukemia. According to statistics, about 80% of adult acute leukemia is AML. The overall situation of the incidence rate of AML is that the incidence rate of AML in developed countries is higher than that in developing countries and that in western countries is higher than that in eastern countries. Over the world, the annual incidence rate is 2.25/100 000 by population, and the incidence rate increases with age. It is about 1/100 000 under the age of 30 and as high as >15/100 000 over the age of 75. According to statistics, the overall annual incidence rate of AML in China is 1.62/100 000, and the incidence rate increases with age. It starts to rise significantly at the age of 50, and reaches a peak at the age of 60-69. The incidence rate for male is significantly higher than that for female. In contrast, the overall incidence rate of AML in Europe and the United States is 3-5/100 000, and the mortality rate is 2.8/100 000. In adults, the median age of onset is 65-70 years, and the incidence rate in patients over 65 years is 15.3/100 000-18.1/100 000. The number of males is also significantly greater than the number of females in the affected population, with a ratio of approximately 5:3.

The treatment strategy for AML has not changed much in the past 30 years. At present, the traditional standard regimen for AML is still the inductive remission by "7+3", that is, 7 days of Cytarabine (Ara-C) plus 3 days of anthracycline chemical drugs such as daunorubicin. The complete remission (CR) can be achieved after 1-2 treatment courses. Then a high-dose Ara-C is repeatedly administered for the post-remission treatment, or an allogeneic hematopoietic stem cell transplantation (allo-HSCT) is performed. Because AML is a highly heterogeneous malignancy, 40% of patients fail to achieve CR despite traditional regimens, and the overall survival (OS) is low. Although hematopoietic stem cell transplantation can maximize the treatment of AML, it is limited by factors such as insufficient donor source or lower matching degree of allogeneic hematopoietic stem cell transplantation (allo-HSCT) and economic conditions, which makes the applicable population limited. Moreover, the vast majority of patients will eventually relapse, and the five-year survival rate is about 40-50%. Therefore, the medical community is eager to find and develop new targets and new drugs for AML.

With the continuous development of gene sequencing technology, researchers have found that more and more gene mutations play an important role in the pathogenesis of AML. Studies have shown that in all AML patients, about one-third of patients have FLT3 mutations, and the main mutation type is FLT3-ITD (internal tandem duplication) mutation. In addition, more than 70% of blast cells (Blasts) of AML patients also showed high expression of FLT3. Clinical studies have shown that patients with FLT3 mutation or high expression of FLT3 also have poor prognosis. AML patients with FLT3-mutation generally suffer from relapse, low OS, and overall unfavourable prognosis after standard chemotherapy, which limits the therapeutic effect of chemotherapy drugs. Since the FLT3 mutation is one of the pathogenesis of AML, FLT3 has become an important target for the development of small molecule targeted drugs for the treatment of AML.

On April 28, 2017, Midostaurin (Trade name: Rydapt), an FLT3 inhibitor from Novartis Pharmaceuticals Corp., USA, was approved by the FDA for marketing, and was used in combination with chemotherapy to treat newly treated patients with FLT 3-positive AML. The clinical study showed that the group receiving Rydapt in combination with chemotherapy had a significant improvement in overall survival compared to the control group receiving chemotherapy alone, with the death risk being reduced by 23%. In addition, the median event-free survival for patients receiving chemotherapy was only 3.0 months, compared with 8.2 months for patients receiving the combination therapy, showing a statistically significant improvement. Subsequently, Gilteritinib (trade name: XOSPATA) of Astellas Pharma and the oral FLT3 inhibitor Quizartinib (trade name: Vanflyta) developed by Daiichi Sankyo were marketed one after another for the treatment of patients having FLT3⁺Relapsed or refractory AML and Relapsed/refractory (R/R) AML with FLT3-ITD mutation respectively. In addition, several other AML therapeutic drugs targeting FLT3 mutations are in clinical trials.

Although it is generally believed that the adverse reactions of targeted drugs will be significantly less than those of traditional chemotherapy drugs, different degrees of toxic and side effects are still observed during clinical trials of FLT3 inhibitors. According to reports, the common adverse reactions of Rydapt in the treatment of AML mainly include leukopenia, nausea and vomiting, mucositis, headache, skin petechiae, musculoskeletal pain, epistaxis, hyperglycemia, upper respiratory tract infection, and the like. The most common non-hematological serious adverse reactions reported in Gilteritinib clinical trials include pneumonia, sepsis, fever, dyspnea and renal damage, and the like. Some patients even permanently discontinue the Gilteritinib treatment due to these serious adverse reactions. Common adverse reactions such as nausea (38%), anemia (29%), QT prolongation (26%), vomiting (26%), febrile neutropenia (25%), diarrhea (20%) and fatigue (20%) are also observed during the phase II clinical trial of oral Quizartinib in the treatment of relapsed AML, refractory AML to second-line therapy, or AML relapsed after hematopoietic stem cell transplantation, and the dose-limiting toxicity response (namely QT prolongation of Grade 3) occurs, suggesting that the drug has obvious cardiotoxicity. During the study, 10% of patients discontinue the treatment due to adverse events. Therefore, the development of safe and effective AML drugs targeting FLT3 mutations still provides a direction for the related research efforts.

Compound A is a novel multi-target protein kinase inhibitor with the following structure:

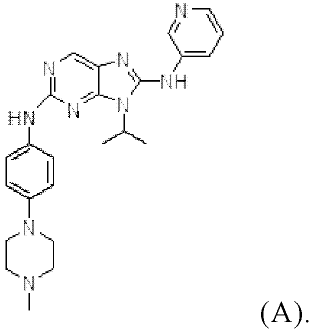

In vitro studies have shown that the main targets of compound A include FLT3, EGFR, Abl, Fyn, Hck, Lck, Lyn, Ret, Yes, and the like, and can be used for the treatment of the above kinase-related tumors. However, there is no study to investigate its efficacy and safety in the treatment of human AML and other tumors, especially AML with FLT3-ITD (internal tandem duplication) mutation.

### Summary of the Invention

In view of the above-mentioned defects in the prior art, one of the objects of the present invention is to provide use of a multi-target protein kinase inhibitor compound A or a pharmaceutically acceptable salt thereof in the manufacture of a medicament for the treatment of tumors, especially leukemia, especially human leukemia.

In some embodiments, the leukemia is acute myeloid leukemia (AML).

In some embodiments, the acute myeloid leukemia (AML) is relapsed and/or refractory acute myeloid leukemia. The relapsed AML refers to relapse following CR, which is defined as reappearance of leukemic blasts in the peripheral blood or the finding of more than 5% blasts in the BM, not attributable to another cause (eg, BM regeneration after consolidation therapy) or extramedullary relapse. The refractory AML includes the preliminarily diagnosed patients who have not achieved ideal curative effect after 2 courses of standard regimen; those relapsed within 12 months who have undergone consolidation and intensive therapy after CR; those who have relapsed after 12 months and have no remission after conventional chemotherapy; those who have relapsed for twice or more times; and those with persistent extramedullary leukemia.

In some embodiments, the relapsed and/or refractory acute myeloid leukemia refers to the relapsed and/or refractory AML with treatment failure with one or more of drugs such as daunorubicin, idarubicin, cytarabine, azacitidine, fludarabine, decitabine, granulocyte colony stimulating factor (G-CSF), homoharringtonine, mitoxantrone, etoposide, and Type II FLT3 inhibitor.

In some embodiments, the AML comprises AML with FLT3-ITD mutation and/or FLT3-TKD (tyrosine kinase domain) mutation, relapsed and/or refractory AML with treatment failure with a Type II FLT3 inhibitor, or DEK-CAN positive AML with FLT3-ITD mutation. Preferably, the Type II FLT3 inhibitor is sorafenib.

In some embodiments, the AML is AML with FL T3-ITD^{high} mutation.

In some embodiments, the FAB classification of the AML is subtype M2, M4, or M5, preferably subtype M5.

In some embodiments, the unfavorable prognostic factors of the AML are 0-2.

In some embodiments, compound A or a pharmaceutically acceptable salt thereof can be used in the manufacture of the medicament in combination with one or more of other targeted drugs or chemotherapeutic drugs clinically used for the treatment of tumor-related diseases.

In some embodiments, the medicament is manufactured into a clinically acceptable formulation, for example oral formulation, injection formulation, topical formulation, external formulation, and the like.

In some embodiments, the medicament is a single-dose dosage form or a fractional-dose dosage form. The dosage form contains a therapeutically effective amount of compound A or a pharmaceutically acceptable salt thereof. The therapeutically effective amount is preferably from about 0.001 mg to about 1000 mg, further preferably from about 1 mg to about 500 mg, or from about 20 mg to about 400 mg, or from about 100 mg to about 350 mg, most preferably from about 150 mg to about 330 mg, or from about 160 mg to about 310 mg, or from about 160 mg to about 300 mg.

In some embodiments, the dosage form is a single-dose dosage form of quaque die (once a day), which contains from about 20 mg to about 500 mg, preferably from about 150 mg to about 400 mg, for example about 150 mg, about 160 mg, about 200 mg, about 250 mg, about 300 mg, about 310 mg, about 350 mg, or about 400 mg of compound A or a pharmaceutically acceptable salt thereof.

In some embodiments, the dosage form is a fractional-dose dosage form of bis in die (twice daily), and each fractional-dose contains from about 100 mg to about 300 mg, preferably from about 100 mg to about 200 mg, for example about 100 mg, about 125 mg, about 150 mg, about 175 mg, about 200 mg of compound A or a pharmaceutically acceptable salt thereof.

Another object of the present invention also includes providing a safe and effective dose of a multi-target protein kinase inhibitor compound A or a pharmaceutically acceptable salt thereof for treating AML, especially human AML, and providing a method of treating AML, particularly human AML. The method comprises administering to a subject or a patient a therapeutically effective amount of compound A or a pharmaceutically acceptable salt thereof. The administering can be oral administration, injection administration, local administration, or external administration. The therapeutically effective amount can treat or alleviate the AML in the subject or the patient.

In some embodiments, the acute myeloid leukemia (AML) is relapsed and/or refractory acute myeloid leukemia. The relapsed AML refers to relapse following CR, which is defined as reappearance of leukemic blasts in the peripheral blood or the finding of more than 5% blasts in the BM, not attributable to another cause (eg, BM regeneration after consolidation therapy) or extramedullary relapse. The refractory AML includes the preliminarily diagnosed patients who have not achieved ideal curative effect after 2 courses of standard regimen; those relapsed within 12 months who have undergone consolidation and intensive therapy after CR; those who have relapsed after 12 months and have no remission after conventional chemotherapy; those who have relapsed for twice or more times; and those with persistent extramedullary leukemia.

In some embodiments, the relapsed and/or refractory acute myeloid leukemia refers to the relapsed and/or refractory AML with treatment failure with one or more of drugs such as daunorubicin, idarubicin, cytarabine, azacitidine, fludarabine, decitabine, granulocyte colony stimulating factor (G-CSF), homoharringtonine, mitoxantrone, etoposide, and Type II FLT3 inhibitor.

In some embodiments, the AML comprises AML with FLT3-ITD mutation and/or FLT3-TKD mutation, relapsed and/or refractory AML with treatment failure with a Type II FLT3 inhibitor, or DEK-CAN positive AML with FLT3-ITD mutation. Preferably, the Type II FLT3 inhibitor is sorafenib.

In some embodiments, the AML is AML with FL T3-ITD^{high} mutation.

In some embodiments, the FAB classification of the AML is subtype M2, M4, or M5, preferably subtype M5.

In some embodiments, the unfavorable prognostic factors of the AML are 0-2.

In some embodiments, the therapeutically effective amount can be from about 0.001 mg/kg to about 1000 mg/kg; preferably, from about 0.01 mg/kg to about 100 mg/kg omni die (daily). Preferably, compound A or a pharmaceutically acceptable salt thereof is administered in a daily dose of from about 0.001 mg to about 1000 mg, preferably from about 1 mg to about 500 mg, or from about 20 mg to about 400 mg, or from about 100 mg to about 350 mg, more preferably from about 150 mg to about 330 mg, or from about 160 mg to about 310 mg, or from about 160 mg to about 300 mg. It is administered in a single dose or in a fractional dose.

In some embodiments, the treatment method is as follow: compound A or a pharmaceutically acceptable salt thereof is administered quaque die (once daily) in a dosage of from about 20 mg to about 500 mg, preferably from about 150 mg to about 400 mg, for example about 150 mg, about 160 mg, about 200 mg, about 250 mg, about 300 mg, about 310 mg, about 350 mg, or about 400 mg each time.

In some embodiments, the treatment method is as follow: compound A or a pharmaceutically acceptable salt thereof is administered bis in die (twice daily) in a dosage of from about 100 mg to about 300 mg, preferably from about 100 mg to about 200 mg, for example about 100 mg, about 125 mg, about 150 mg, about 175 mg, or about 200 mg each time.

In some embodiments, compound A or a pharmaceutically acceptable salt thereof is manufactured into a clinically acceptable formulation and then administered, and the said formulation comprises oral formulation, injection formulation, topical formulation, external formulation, and the like.

In some embodiments, compound A or a pharmaceutically acceptable salt thereof can be used to treat the disease in combination with one or more of other targeted drugs or chemotherapeutic drugs clinically used for the treatment of tumor-related diseases.

The present invention also provides compound A or a pharmaceutically acceptable salt thereof for use in treating AML, especially human AML.

In some embodiments, the acute myeloid leukemia (AML) is relapsed and/or refractory acute myeloid leukemia. The relapsed AML refers to relapse following CR, which is defined as reappearance of leukemic blasts in the peripheral blood or the finding of more than 5% blasts in the BM, not attributable to another cause (eg, BM regeneration after consolidation therapy) or extramedullary relapse. The refractory AML includes the preliminarily diagnosed patients who have not achieved ideal curative effect after 2 courses of standard regimen; those relapsed within 12 months who have undergone consolidation and intensive therapy after CR; those who have relapsed after 12 months and have no remission after conventional chemotherapy; those who have relapsed for twice or more times; and those with persistent extramedullary leukemia.

In some embodiments, the relapsed and/or refractory AML refers to relapsed and/or refractory AML with treatment failure with one or more of drugs such as daunorubicin, idarubicin, cytarabine, azacitidine, fludarabine, decitabine, granulocyte colony stimulating factor (G-CSF), homoharringtonine, mitoxantrone, etoposide, and Type II FLT3 inhibitor.

In some embodiments, the AML comprises AML with FLT3-ITD mutation and/or FLT3-TKD mutation, relapsed and/or refractory AML with treatment failure with a Type II FLT3 inhibitor, or DEK-CAN positive AML with FLT3-ITD mutation. Preferably, the Type II FLT3 inhibitor is sorafenib.

In some embodiments, the AML is AML with FL T3-ITD^{high} mutation.

In some embodiments, the FAB classification of the AML is subtype M2, M4, or M5, preferably subtype M5.

In some embodiments, the unfavorable prognostic factors of the AML are 0-2.

In some embodiments, compound A or a pharmaceutically acceptable salt thereof can be administered in combination with one or more of other targeted drugs or chemotherapeutic drugs clinically used for the treatment of tumor-related diseases.

In some embodiments, the treatment comprises administering a therapeutically effective amount of compound A or a pharmaceutically acceptable salt thereof, and the therapeutically effective amount is from about 0.001 mg/kg to about 1000 mg/kg; preferably, from about 0.01 mg/kg to about 100 mg/kg per day; preferably, compound A or a pharmaceutically acceptable salt thereof is administered in a daily dose of from about 0.001 mg to about 1000 mg, preferably from about 1 mg to about 500 mg, or from about 20 mg to about 400 mg, or from about 100 mg to about 350 mg, most preferably from about 150 mg to about 330 mg, or from about 160 mg to about 310 mg, or from about 160 mg to about 300 mg; administered in a single dose or in a fractional dose.

In some embodiments, compound A or a pharmaceutically acceptable salt thereof is administered quaque die (once daily) in a dosage of from about 20 mg to about 500 mg, preferably from about 150 mg to about 400 mg each time. For example, about 150 mg, 160 mg, 200 mg, 250 mg, 300 mg, 310 mg, 350 mg, or 400 mg is administered each time; or compound A or a pharmaceutically acceptable salt thereof is administered bis in die (twice daily) in a dosage of from about 100 mg to about 300 mg, preferably from about 100 mg to about 200 mg, for example about 100 mg, about 125 mg, about 150 mg, about 175 mg, or about 200 mg each time.

The present invention also provides a pharmaceutical composition, comprising compound A of the following formula or a pharmaceutically acceptable salt thereof and an optional pharmaceutically acceptable excipient, wherein said pharmaceutical composition is used for treating human AML,

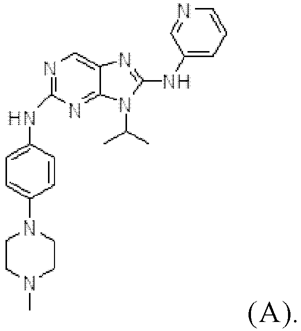

In some embodiments, the acute myeloid leukemia (AML) is relapsed and/or refractory acute myeloid leukemia. The relapsed AML refers to relapse following CR, which is defined as reappearance of leukemic blasts in the peripheral blood or the finding of more than 5% blasts in the BM, not attributable to another cause (eg, BM regeneration after consolidation therapy) or extramedullary relapse. The refractory AML includes the preliminarily diagnosed patients who have not achieved ideal curative effect after 2 courses of standard regimen; those relapsed within 12 months who have undergone consolidation and intensive therapy after CR; those who have relapsed after 12 months and have no remission after conventional chemotherapy; those who have relapsed for twice or more times; and those with persistent extramedullary leukemia.

In some embodiments, the relapsed and/or refractory AML refers to relapsed and/or refractory AML with treatment failure with one or more of drugs such as daunorubicin, idarubicin, cytarabine, azacitidine, fludarabine, decitabine, granulocyte colony stimulating factor (G-CSF), homoharringtonine, mitoxantrone, etoposide, and Type II FLT3 inhibitor.

In some embodiments, the AML comprises AML with FLT3-ITD mutation and/or FLT3-TKD mutation, relapsed and/or refractory AML with treatment failure with a Type II FLT3 inhibitor, or DEK-CAN positive AML with FLT3-ITD mutation. Preferably, the Type II FLT3 inhibitor is sorafenib.

In some embodiments, the AML is AML with FL T3-ITD^{high} mutation.

In some embodiments, the FAB classification of the AML is subtype M2, M4, or M5, preferably subtype M5.

In some embodiments, the unfavorable prognostic factors of the AML are 0-2.

In some embodiments, the pharmaceutical composition can be administered in combination with one or more of other targeted drugs or chemotherapeutic drugs clinically used for the treatment of tumor-related diseases.

In some embodiments, the treatment comprises administering a pharmaceutical composition comprising a therapeutically effective amount of compound A or a pharmaceutically acceptable salt thereof, and the therapeutically effective amount of compound A or a pharmaceutically acceptable salt thereof is from about 0.001 mg/kg to about 1000 mg/kg, preferably, from about 0.01 mg/kg to about 100 mg/kg omni die (daily); preferably, the pharmaceutical composition is administered in a daily dose of from about 0.001 mg to about 1000 mg, preferably from about 1 mg to about 500 mg, or from about 20 mg to about 400 mg, or from about 100 mg to about 350 mg, more preferably from about 150 mg to about 330 mg, or from about 160 mg to about 310 mg, or from about 160 mg to about 300 mg of compound A or a pharmaceutically acceptable salt thereof; administered in a single dose or in a fractional dose.

In some embodiments, the pharmaceutical composition is administered quaque die (once daily) in the dosage of from about 20 mg to about 500 mg, preferably from about 150 mg to about 400 mg, for example about 150 mg, about 160 mg, about 200 mg, about 250 mg, about 300 mg, about 310 mg, about 350 mg, or about 400 mg of compound A or a pharmaceutically acceptable salt thereof each time; or the pharmaceutical composition is administered bis in die (twice daily) in the dosage of from about 100 mg to about 300 mg, preferably from about 100 mg to about 200 mg, for example about 100 mg, about 125 mg, about 150 mg, about 175 mg or about 200 mg of compound A or a pharmaceutically acceptable salt thereof each time.

The dose of the compound A or a pharmaceutically acceptable salt thereof of the present invention is calculated as Compound A.

In the technical solution of the present application, "about" before a numerical value means within ±10% of the numerical value, preferably ±5%, for example, ±10%, ±9%, ±8%, ±7%, 6%, ±5%, ±4%, ±3%, ±2% or ±1% of the numerical value.

In order to evaluate the efficacy and safety of compound A or a pharmaceutically acceptable salt thereof in the treatment of human AML, especially AML with FLT3 mutation, the inventors of the present application conducted clinical trials of phase I and phase II. Preliminary study results show that compound A has certain efficacies both on treating AML with FLT3-ITD mutation and/or FLT3-TKD mutation, and DEK-CAN positive AML with FLT3-ITD mutation. Patients with relapsed and/or refractory AML who have failed treatment with a Type II FLT3 inhibitor (e.g., sorafenib) can still clinically benefit from the treatment with compound A. A low incidence of ADR ≥ grade 3 was detected in clinical trials of compound A or a pharmaceutically acceptable salt thereof, and no serious toxic responses in the heart, liver and blood system were observed. Therefore Compound A exhibits good clinical safety.

Preliminary results of the clinical trial of phase II showed that (the groups of) 150 mg BID and 300 mg QD of compound A had tolerable safety in treating the relapsed and/or refractory AML with FLT3 mutation, and the groups of 150 mg BID and 300 mg QD both showed a certain clinical benefit and were better than the group of 200 mg BID in the efficacy and the safety. The preliminary observation results showed that Compound A was comparable to similar products Quizatinib and Gilteritinib in the efficacy, and had a significantly reduced incidence of serious cardiac, hepatic and hematological adverse reactions. It is suggested that compound A is expected to treat AML safely and effectively.

### Brief description of the drawings

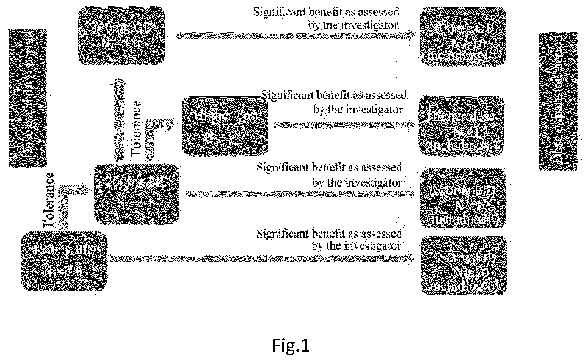

Figure 1 is a schematic diagram of the Phase II clinical study method.

### Detailed description of the invention

The following examples are for the purpose of better illustrating the content of the present invention, but the content of the present invention is not limited to the examples. Non-essential improvements and adjustments to the embodiments made by those skilled in the art according to the above-mentioned contents of the invention still belong to the protection scope of the present invention.

### 1. Phase I clinical study

### 1.1 Study objectives

To investigate the safety/tolerability, pharmacokinetic characteristics and preliminary efficacy of single and multiple administrations of compound A in the subjects with FLT3-mutant AML, and to provide the basis for the design of phase II clinical trial of compound A.

### 1.2 Study method

### 1.2.1 Case inclusion criteria

The case inclusion criteria used in this study were as follows:
(1) Inclusion criteria:
   1) Voluntarily underwent the test and written informed consent forms;
   2) Male or female Chinese patients, age ≥ 18 years;
   3) Diagnosed patients with primary or secondary AML conforming to the classification of the World Health Organization (WHO), who were positive for the FLT3 mutation by the leukemia cell gene detection and fulfilled one of the following:
      a) Refractory to at least one cycle of the induction treatment; or
      b) Relapsed after achieving remission with a prior therapy; or
      c) Preliminarily diagnosed patients that were judged by the investigator to be unsuitable for the induction chemotherapy due to age, comorbidity or other reasons;
   4) Eastern Cooperative Oncology Group (ECOG) performance status of 0-3;
   5) Before the subject's disease has not progressed rapidly, subject's interval from prior treatment to time of experimental drug administration should be at least 2 weeks for cytotoxic agents (except hydroxyurea), or at least 5 half-lives for noncytotoxic agents;
   6) Serum creatinine ≤ 1.5x institutional upper limit of normal (ULN);
   7) Total bilirubin ≤ 1.5 x institutional ULN unless considered due to Gilbert's syndrome or leukemic organ involvement;
   8) Serum AST and ALT ≤ 3.0 x institutional ULN unless considered due to leukemic organ involvement;
   9) Subjects of childbearing age agree to take effective contraceptive measures during treatment and 6 months after the final administration of experimental drug.
(2) Exclusion Criteria:
   1) Diagnosed as acute promyelocytic leukemia;
   2) Subject has clinically active central nervous system leukemia;
   3) There was a non-hematological toxicities of Grade 2 or higher caused by the prior AML treatment;
   4) Bone marrow transplantation within 100 days prior to the study;
   5) Uncontrollable active infections;
   6) Major surgery on major organ within 4 weeks prior to the study;
   7) Radiotherapy performed within 4 weeks prior to the study;
   8) Cardiac ejection fraction lower than 50% or lower than the lower limit of normal; patients with a history of clinically significant QTc prolongation (male > 450ms, female > 470ms); a history of severe heart disease;
   9) HIV positive;
   10) Active hepatitis B or C, or other hepatic disorder;
   11) Pregnant or lactating women;
   12) Serious Medical condition or intercurrent illness, or other extenuating circumstance that, in the judgment of the Investigator, could jeopardize patient safety or interfere with the objectives of the study.
   13) Patients incapable of entering the study in the investigator's opinion.

### 1.2.2 Efficacy evaluation standard

It was established with reference to "*Hematopathy Diagnosis and Therapeutic Effect Standards* (Third Edition) ", and detailed as follows:
1. Remission standard:
   (1) Complete remission (CR):
      1) In clinic, there were no symptoms and signs caused by leukemia cell infiltration, and the life was normal or close to normal.
      2) Hemogram: Hb ≥ 100g/L (male) or 90g/L (female and children), the absolute neutrophil count ≥ 1.5 × 10⁹/L, and the platelet count ≥ 100 × 10⁹/L. Absence of leukemic blasts in the peripheral blood by morphological examination.
      3) Myelogram: myeloblasts type I + type II (primitive monocytes + immature monocytes or primitive lymphocytes + immature lymphocytes) ≤ 5%, erythrocytes and megakaryocytes were both normal.
         M2b type: myeloblasts type I + type II ≤ 5%, the proportion of neutrophilic myelocytes was in the normal range.
         M3 type: myeloblasts + promyelocytes ≤ 5%.
         M4 type: myeloblasts types I and II + primitive monocytes and immature monocytes ≤5%.
         M5 type: primitive monocytes type I + type II and immature monocytes ≤5%.
         M6 type: myeloblaststype I + type II≤5%, the proportion of rubriblasts and normoblasts was substantially normal.
         M7 type: The proportion of granulocytes and erythrocytes was normal, and primitive megakaryocytes and immature megakaryocytes basically disappeared.
         Acute lymphoblastic leukemia: primitive lymphocytes + immature lymphocytes ≤ 5%.
   (2) Partial remission (PR): medullary myeloblasts type I + type II (primitive monocytes + immature monocytes or primitive lymphocytes + immature lymphocytes) >5% but ≤20%, and failing to meet the standard for CR in one of the clinical items or the hemogram items.
2. Relapse of leukemia: it was referred to as relapse if one of the following three conditions occured after CR was obtained through the treatment.
   (1) Medullary myeloblasts type I + type II (primitive monocytes +immature monocytes or primitive lymphocytes + immature lymphocytes) >5% but ≤20%, and failing to meet the standard on the myelogram for CR after one course of the effective AML treatment.
   (2) Medullary myeloblasts type I + type II (primitive monocytes +immature monocytes or primitive lymphocytes + immature lymphocytes) >20%.
   (3) The occurrence of extramedullary disease.
3. Continued complete remission (CCR): meaning no leukemia recurrence for more than 3-5 years from the date of CR after the treatment.
4. Long-term survival: survival (including disease-free survival or disease survival) for 5 years or more from the date of diagnosis of acute leukemia.
5. Clinical cure: stopping chemotherapy for 5 years or disease-free survival (DFS) for 10 years.

Note: The statistics of survival rate should include those with less than one course of the induction therapy; the cases with one course of induction therapy or more should be included in the scope of efficacy statistics.

### 1.2.3 Treatment methods

The study included AML subjects aged ≥18 years with primary or secondary AML who were positive for the FLT3 mutation. The test was divided into 8 groups, and the administration dosages were 20mg, 40mg, 80mg, 120mg, 160mg, 200mg, 250mg, 310mg (as compound A).

Each subject firstly underwent a single-administration study on tolerability and pharmacokinetics on day 1 (i.e., d1) followed by a multiple-administration study on tolerability and pharmacokinetics on day 4 (d4). It was administered in 28-day cycles for the multiple-administration. After each subject completed the first cycle of the study on tolerability and pharmacokinetics and if there was neither dose-limiting toxicity nor disease progression, it was up to the investigator to determine whether the subject could continue the treatment, until the disease progressed or the intolerable toxicity appeared. The "dose-limiting toxicity (DLT)" was defined as follows: according to the NCI-CTCAE v4.0, within the first cycle (28 days) of the multiple-administration stage, the occurrence of non-hematological toxicity of grade 3 and above likely associated with (including definitely related to, probably related to and possibly related to) compound A was defined as dose-limiting toxicity.

The study adopted a "3+3" design. During the study, if no DLT occurred in the first cycle of a certain dose group, the dose escalation continued. If one subject out of a certain dose group experienced the DLT in the first cycle, three more subjects should be added to this dose group; if one or more out of these three subjects experienced the DLT, the trial would be terminated. If two or more subjects out of a certain dose group experienced the DLT in the first cycle, the trial would be terminated, and the dose just preceding this dose would be regarded as MTD. After the MTD was determined, three more subjects were enrolled for observation in the dose group where the MTD was located.

### 1.3 Study results

A total of 33 subjects were screened in the trial, 5 subjects failed the screening, 28 subjects took drugs, and 2 subjects dropped out. Twenty-eight subjects accepted at least one administration, one subject for 4 days, one subject for 7 days, and all the remaining subjects for at least 28 days.

### 1.3.1 Baseline characteristics of subjects

A total of 28 enrolled subjects were screened, of which 14.29% were the relapsed AML patients, 57.14% were the refractory patients, and 28.57% were the relapsed/refractory AML patients. 96.43% of the subjects had FLT3-ITD mutations, and the median age was 50 years (19-70 years) (see Table 1 for details).

**Table 1: Baseline characteristics of subjects**

| Characteristics | N (%) or median (range) |
|---|---|
| Number of patients | 28(100.00) |
| Age | 50(19-70) |
| Gender | |
| Male | 11(39.29) |
| Female | 17(60.71) |
| Ethnic group | |
| Han | 26(92.86) |
| Others | 2(7.14) |
| ECOG performance status | |
| 0 | 3(10.71) |
| 1 | 12(42.86) |
| 2 | 9(32.14) |
| 3 | 4(14.29) |
| Diagnosis | |
| Relapsed AML | 4(14.29) |
| Refractory AML | 16(57.14) |
| Relapsed/refractory AML | 8(28.57) |
| Subtype | |
| M1 | 9(32.14) |
| M2 | 9(32.14) |
| M4 | 8(28.58) |
| M5 | 2(7.14) |
| FLT3 status | |
| ITD | 27(96.43) |
| Others | 1(3.57) |
| Number of prior treatment regimens | 3.50(1-9) |

### 1.3.2 Safety

During the trial, the dose-limiting toxicity was observed in one subject in the 160 mg dose group, and it was the elevation in ALT and AST. Subsequently, three subjects were added to this group, and DLT did not reemerge in the three additional subjects. The dose escalation continued until no MTD was detected at 310 mg QD.

### (1) Common adverse reactions

Common adverse reactions occurred during the study included diarrhea (7 cases, 13 times), rash (4 cases, 9 times), increased total protein (2 cases, 5 times), decreased WBC (1 case, 5 times), and the like. The adverse reactions of grade 3 or above (6 cases) were mainly decreased WBC, decreased granulocytes, bone marrow function failure, abnormal liver function, diarrhea, and the like. All improved after the symptomatic treatment. The incidence of AEs of grade 3/4 had no significant difference between the first treatment cycle and the subsequent treatment cycles (see Table 2).

**Table 2: The occurrence of adverse events of grade 3/4 in the first treatment cycle and the subsequent treatment cycles**

| | The first treatment cycle | | | | | | The second and subsequent treatment cycles | | | | | |
|---|---|---|---|---|---|---|---|---|---|---|---|---|
| SOC | Grade 3 | | | Grade 4 | | | Grade 3 | | | Grade 4 | | |
| PT | Number of cases | Percent | Number of times | Number of cases | Percent | Number of times | Number of cases | Percent | Number of times | Number of cases | Percent | Number of times |
| Total | 5 | 27.78 | 8 | 2 | 11.11 | 4 | 8 | 44.44 | 40 | 3 | 16.67 | 4 |

### (2) SAEs that may not be related to the drug

There were a total of 13 subjects in the trial who developed SAEs that may not be related to the drug, including 4 cases of AML progression, 6 cases of infection-related SAEs, 3 cases of bleeding-related SAEs, 1 case of incomplete intestinal obstruction, 1 case of disease-related death, and 1 case of fever.

### (3) SAEs that may be related to the drug

There was only one patient in the trial who developed a potentially drug-related SAE, namely, nephrotic syndrome, total cardiac failure, and pulmonary hypertension occured in the 14th cycle.

### 1.3.3 Preliminary validity

All 28 subjects were administered at least once, of which one subject withdrew due to his/her own reason only after 4 days of the administration, one subject developed DLT (with multiple administrations for 7 days and then discontinued), and all the other subjects were administered for at least 28 days, and the overall response rate (ORR) was 21.4% (6/28). This study was a dose-escalation study, and the 160mg group began to show the efficacy. There were a total of 16 subjects in the 160mg, 200mg, 250mg, and 310mg dose groups, of which one subject was in CR and five subjects were in partial remission, showing the preliminary efficacy. Therefore, the effective rate above the effective dose was 37.5% (6/16). One subject in the 250 mg group was assessed as CR and one subject in the 310 mg group achieved bone marrow CR.

In addition, there were hematologic improvement cases just from the 20 mg group, specifically: 13 subjects of hematologic improvement in platelet (HI-P) and 12 cases of hematologic improvement in neutrophil (HI-N).

There were 14 subjects in this study who had previously received the sorafenib treatment, but did not achieve the remission or experienced the disease recurrence. Among them, one subject was in CR after the treatment with compound A (250 mg QD), and one subject was in partial remission. It was suggested that the treatment with compound A could still benefit patients with R/R AML after failure of sorafenib treatment.

Some studies showed that DEK-CAN positivity coexisted with FLT3-ITD mutation, which was associated with a lower response rate and shorter survival in AML. In this trial, there were a total of 2 subjects having both FLT3-ITD mutation and DEK-CAN positivity. Among others, one subject achieved CR after 2 cycles of compound A (250 mg QD) treatment and was still receiving the treatment; and one subject achieved PR after 2 cycles of the treatment. It was suggested that compound A still showed good efficacy for AML patients having both DEK-CAN positivity and FLT3-ITD mutation.

**Table 3: Evaluation of best response to tumor in subjects**

| | | FAS (N=28) | | | | | | | | |
|---|---|---|---|---|---|---|---|---|---|---|
| Index | | 20 mg group (N=3) | 40 mg group (N=3) | 80 mg group (N=3) | 120 mg group (N=3) | 160 mg group (N=3) | 200 mg group (N=7) | 250 mg group (N=3) | 310 mg group (N=3) | Total |
| Evaluation of best response | | | | | | | | | | |
| | CR | 0 | 0 | 0 | 0 | 0 | 0 | 1 | 0 | 1 |
| | PR | 0 | 0 | 0 | 0 | 1 | 3 | 0 | 1 | 5 |
| | No response | | | | | | | | | |
| | SD | 3 | 3 | 3 | 3 | 2 | 2 | 2 | 2 | 20 |
| | PD | 0 | 0 | 0 | 0 | 0 | 0 | 0 | 0 | 0 |
| | NE | 0 | 0 | 0 | 0 | 0 | 2 | 0 | 0 | 2 |
| | Total | 3 | 3 | 3 | 3 | 3 | 7 | 3 | 3 | 28 |

| Overall response rate | | | | | | | | | | |
|---|---|---|---|---|---|---|---|---|---|---|
| | ORR | 0 | 0 | 0 | 0 | 0.33 | 0.43 | 0.33 | 0.33 | 0.21 |

### 1.4 Comparison with similar products

Referring to the clinical study results of the first-generation and second-generation FLT3 inhibitors disclosed in the prior art, and comparing the differences in the efficacy and safety of compound A of the present invention and the existing FLT-3 inhibitors, it could be found that the preliminary efficacy of compound A, as shown in the clinical trial of Phase I, was comparable to those of the existing second-generation FLT3 inhibitors (Quizartinib or Gilteritinib), and remarkably better than those of the first-generation FLT3 inhibitors (Sorafenib or Midostaurin). Moreover, the main toxicity of compound A was mild and mainly diarrhea and rash. It had relatively high clinical application value.

**Table 4: Comparison of compound A with similar products**

| FLT-3 inhibitors | | Non-FLT3 targets | FLT3-TKD mutation activity | CR rates in monotherapy for R/R AML | Dose | Main toxicity | Approved status |
|---|---|---|---|---|---|---|---|
| First generation | Sorafenib | c-KIT, PDGFR, RAF, VEGFR | No | <10% | 400 mg BID | Rash, bleeding, myelosuppression | Off-label (approved by FDA for liver cancer, kidney cancer, and differentiated thyroid cancer) |
| | Midostaurin | c-KIT, PKC, PDGFR, VEGFR | Yes | <10% | 50 mg BID | Gastrointestinal toxicity, and myelosuppression | Approved by FDA and EMA for treatment of newly diagnosed FLT3-mutant AML in combination with chemotherapy |
| Second-generation | Quizartinib | c-KIT, PDGFR, RET | No | 24-47% | 30-60 mg QD | QT interval prolongation, and myelosuppression | Approved for R/R AML in Japan; and not approved by FDA and EMA |
| | Gilteritinib | AXL | Yes | 37-41% | 120 mg QD | Transaminase elevation, and diarrhea | Conditional approval for R/R AML by FDA in 2018 |
| | Compound A | EGFR, Abl, Fyn, Hck, Lck, Lyn, Ret, Yes, and the like | No | 33.3% | 250 mg -310 mg QD | Diarrhoea and rash | Clinical Phase IIa |

### 1.4.1 Comparison of grade ≥3 ADRs

In the phase II clinical trial of gilteritinib, febrile neutropenia and anemia of grade ≥3 occured in nearly half of the subjects. Whereas, in the above-mentioned phase I clinical trial of compound A, such adverse reactions seldom occurred.

**Table 5 ADRs of grade ≥ 3 of compound A**

| ADRs of grade ≥ 3 | Compound A (all dose groups, N=28) |
|---|---|
| Leukocytopenia | 1 (3.6) |
| Elevated serum creatinine | 1 (3.6) |
| Neutropenia | 1 (3.6) |
| Hyperuricemia | 1 (3.6) |
| Abnormal liver function | 1 (3.6) |
| Diarrhoea | 1 (3.6) |
| Tachypnea | 1 (3.6) |
| Discomfort | 1 (3.6) |
| Fever | 1 (3.6) |
| Fatigue | 1 (3.6) |
| Peripheral edema | 1 (3.6) |
| Bone marrow failure | 1 (3.6) |
| Agranulemia | 1 (3.6) |

**Table 6 ADRs of grade ≥ 3 of Gilteritinib**

| Most common ADRs of grade ≥ 3 | Gilteritinib (120 mg daily, N=246) |
|---|---|
| Febrile neutropenia | 113 (45.9%) |
| Anemia | 100 (40.7%) |
| Thrombocytopenia | 56 (22.8%) |

| | |
|---|---|
| (Note: Data source: Gilteritinib phase II-ADMIRAL trial) | |

### 1.4.2 Cardiotoxicity comparison

Different degrees of cardiotoxicity reactions were observed during clinical studies of Gilteritinib and Quizartinib. The results of the Phase I clinical trial showed that the incidence of the cardiotoxicity of compound A of the present invention was low, the degree thereof was mild, and no cardiotoxicity of grade ≥3 occurred.

**Table 7: Cardiotoxicity of compound A**

| ADR | Compound A (all dose groups, N=28) |
|---|---|
| | Number of cases (%) |
| QT interval prolongation | 2 (7.2) |
| Abnormal ST segment | 1 (3.6) |
| Pericardial effusion | 1 (3.6) |

**Table 8: Cardiotoxicity of Gilteritinib**

| ADR | Gilteritinib (120 mg daily, N=292) |
|---|---|
| QT interval prolongation | 8.4% |
| Cardiac failure | 4% |
| Pericardial effusion | 3% |
| Pericarditis | 2% |

**Table 9: Cardiotoxicity of Quizartinib**

| Serious cardiac adverse reaction | Quizartinib (60 mg daily, n=241) |
|---|---|
| QT interval prolongation | 26.6% |
| Myocardial infarction | 0.4% |

### 2. Phase II clinical study

### 2.1 Study objectives

To evaluate the efficacy and safety of Compound A in Chinese adult subjects with FLT3-mutant R/R AML, and to explore the optimal dosage regimen.

### 2.2 Study method

### 2.2.1 Overall study design and plan

This trial was a two-stage, multi-dose escalation, and cohort expansion dynamic design. Based on the analysis of the data of the preceding dose group, it would be decided whether to adjust the dose or expand the subsequent trial. The expansion included the expansion for scaling up/reducing the dose group, the expansion for the subject number, the expansion for the treatment cycle, and the like.

The first stage was the safety and tolerability investigation period. The subjects were divided into 150 mg BID group, 200 mg BID group and 300 mg QD group, 3 subjects in each group. 28 consecutive days was regarded as one treatment cycle. After the first cycle of tolerability and pharmacokinetics studies, if the dose-limiting toxicity (DLT) did not occur or the disease did not progress, it was up to the investigator to judge whether or not the treatment could be continued until the disease progressed or the intolerable toxicity occurred. If one subject out of a certain dose group experienced the DLT in the first cycle, three more subjects should be added to this dose group; if one or more out of these three subjects experienced the DLT, the trials for this dose group and higher dose group(s) would be terminated. If two or more subjects out of a certain dose group experienced the DLT in the first cycle, the trials for this dose group and higher dose group(s) would be terminated.

The trial started with the 150 mg BID group, and after completing the first cycle of the tolerability investigation of 3 subjects in this group, the tolerability study of the 200mg BID group was carried out. The principle of the subject enrollment was to give priority to meeting three subjects in the tolerability investigation period. Since the 300mg QD group had been proven to be well tolerated in a single tolerability trial, other qualified subjects screened during the tolerability investigation period could directly enter the 300mg QD group.

After the 150mg BID group and the 200mg BID group completed the tolerability investigation, investigators, sponsors, quantitative pharmacology experts, clinical pharmacology experts, and statistical experts discussed whether to continue the exploration of higher effective doses.

The second stage was the dose expansion period. After the completion of the first cycle of the study in a dose group, if the investigator assessed that the subjects had significant benefits, the dose group would undergo the cohort expansion to further explore possible effective doses, and continue to increase to at least 10 subjects. During this period, investigators, sponsors, quantitative pharmacology experts, clinical pharmacology experts, and statistical experts discussed whether to continue the cohort expansion according to the efficacy of the dose group (see Figure 1 for the specific trial method).

### 2.2.2 Selection of study population

The inclusion and exclusion criteria used in the phase II clinical study were the same as those used in the phase I clinical study.

### 2.2.3 Validity and safety variables

### 2.2.3.1 Validity variables

Overall CR (CR, CRh, CRi) rate: defined as the proportion of subjects who achieved CR, CRh, CRi during Compound A treatment among all subjects enrolled in this study. Subjects who were not assessed for response were included in the denominator when calculating the response rate. Among others, CR was defined as obtaining a morphologically leukemia-free state, in which neutrophil count ≥ 1 × 10⁹/L, platelets ≥100×10⁹/L, disengagement from the blood transfusion, and the hemoglobin concentration or the hematocrit did not affect the response state; CRi was defined as completely meeting all CR standard except for neutropenia (<1×10⁹/L) or thrombocytopenia (<100×10⁹/L); and CRh was defined as completely meeting all CR standard except for neutropenia (>0.5×10⁹/L, <1×10⁹/L) or thrombocytopenia (>50×10⁹/L, <100×10⁹/L).
(1) Three year overall survival (OS): the OS time was referred to the time from the first drug administration to the death from any cause within 3 years.
(2) Three year event-free survival (EFS): it was referred to the time from the randomized enrollment to the first occurrence of any of the following conditions within 3 years: ① no CR (CR, CRh, CRi) occurred within 6 months of treatment, ② relapsed after reaching the specified CR (CR, CRh, CRi), ③ disease progression, ④ all-cause death at any time during the study, whichever occurred first. The censoring rules for EFS in the trial were: the subjects without baseline myelogram and blood routine assessment and without validity assessment after baseline were censored on the enrollment date; the subjects who withdrew, or had no disease progression or did not die in the trial, or whose conditions about whether or not relapsed, failed the treatment or died were unavailable, were censored on the date of the last efficacy assessment; and the subjects who had received a new anti-leukemia treatment before the disease progression, were censored on the date of the last efficacy assessment before receiving the new anti-leukemia treatment.
(3) Duration of response-CR (DoR-CR): the DoR-CR time was referred to the time from the first achievement of CR to the recording of hematological relapse, extramedullary disease, or death from any cause (whichever was earlier).

### 2.2.3.2 Safety variables

Safety variables included physical examination, vital signs, ECOG performance status, laboratory assessment, adverse events, and the like.

### 2.3 Study results

### 2.3.1 Analytical data sets, demographics, and other baseline characteristics

As of July 15, 2020, a total of 31 subjects were enrolled, and all subjects were included in the FAS set and the SS set. Among them, 14 subjects were enrolled in the 150 mg BID group, 4 subjects were enrolled in the 200 mg BID group, and 13 subjects were enrolled in the 300 mg QD group. Subject demographics and baseline characteristics were shown in Table 10.

Only 1 of the enrolled subjects had previously received an allo-HSCT treatment (CRi at C2 assessment, CRh at C3 assessment, and CR at C4 assessment). All subjects had received the drug treatment in the past for an average of 5.6 cycles, and the median treatment cycle number was 5. Among them, 24 (77.4%) subjects had the best previous response of CR.

During the trial, the subjects had good medication compliance, and most subjects could take the drugs on time and according to the dosage (29 cases, 93.5%). For two subjects who did not take the drugs on time or according to the dosage, their medication compliance was also in the range of 80%-120%.

**Table 10: Subject demographics and baseline characteristics (FAS)**

| | | 150mgBID(n=14) | 200 mg BID (n=4) | 300 mg QD (n=13) | Total (n=31) |
|---|---|---|---|---|---|
| Age | | | | | |
| | A verage±SD | 39.1±12.63 | 54.8±17.35 | 51.1±18.85 | 46.2±16.87 |
| | Median (min, max) | 35.5 (18, 72) | 62.0 (29, 66) | 56.0 (18, 73) | 44.0 (18, 73) |
| Age category, n (%) | | | | | |
| | <65 | 13 (92.9) | 3 (75.0) | 8 (61.5) | 24 (77.4) |
| | ≥65 | 1 (7.1) | 1 (25.0) | 5 (38.5) | 7 (22.6) |
| Gender, n (%) | | | | | |
| | Male | 9 (64.3) | 1 (25.0) | 6 (46.2) | 16 (51.6) |
| | Female | 5 (35.7) | 3 (75.0) | 7 (53.8) | 15 (48.4) |
| BMI | | | | | |
| | A verage±SD | 21.24±4.499 | 25.25±5.482 | 21.42±4.379 | 21.83±4.609 |
| | Median (min, max) | 22.60 (19.2, 32.1) | 23.65 (20.6, 33.1) | 21.70 (13.9, 28.7) | 22.55 (13.9, 33.1) |
| Ethnic group, n (%) | | | | | |
| | Han | 12 (85.7) | 3 (75.0) | 13 (100.0) | 28 (90.3) |
| | Others | 2 (14.3) | 1 (25.0) | 0 (0) | 3 (9.7) |
| ECOG performance status, n (%) | | | | | |
| | 0-1 | 11 (78.6) | 1 (25.0) | 10 (76.9) | 22 (71.0) |
| | 2-3 | 3 (21.4) | 3 (75.0) | 3 (23.1) | 9 (29.0) |
| FLT3 mutation, n (%) | | | | | |
| | ITD | 10 (71.4) | 3 (75.0) | 9 (69.2) | 22 (71.0) |
| | TKD | 4 (28.6) | 1 (25.0) | 4 (30.8) | 9 (29.0) |
| ITD&TKD | | 0(0) | 0(0) | 0(0) | 0(0) |
| AML Type, n (%) | | | | | |
| Refractory | | 3 (21.4) | 0(0) | 2 (15.4) | 5 (16.1) |
| Relapsed | | 6 (42.9) | 2 (50.0) | 8 (61.5) | 16 (51.6) |
| Relapsed and refractory | | 5 (35.7) | 2 (50.0) | 3 (23.1) | 10 (32.3) |
| FAB classification, n (%) | | | | | |
| M1 | | 0(0) | 0(0) | 1 (7.7) | 1 (3.2) |
| M2 | | 3 (21.4) | 0(0) | 3 (23.1) | 6(19.4) |
| M3 | | 0(0) | 0(0) | 0(0) | 0(0) |
| M4 | | 5 (35.7) | 1 (25.0) | 3 (23.1) | 9 (29.0) |
| M5 | | 5 (35.7) | 3 (75.0) | 6 (46.2) | 14 (45.2) |
| M6 | | 0(0) | 0(0) | 0(0) | 0(0) |
| Non-M3 | | 1 (7.1) | 0(0) | 0(0) | 1 (3.2) |

### 2.3.2 Pharmacokinetic studies

After the subjects were orally administered with Compound A for 28 consecutive days, the 150 mg BID group had a similar exposure level and AUC to the 300 mg QD group, while a Cₘₐₓ 50% lower than that of the 300 mg QD group, suggesting a probably better safety. The exposure of the 200 mg BID group was about twice that of the 150 mg BID group, and the early dose reduction was often occured due to adverse reactions, resulting in a relatively poor efficacy. By comprehensive consideration, the first dose-expansion was terminated in the 200 mg BID group. With the increase of the administration frequency, the accumulation degree increased; the accumulation degree of metabolites was slightly higher than that of the original drug. Covariates such as gender, age, and body weight had no significant effect on the PK of compound A.

### 2.3.3 Validity/efficacy assessment

### 2.3.3.1 Overall best efficacy analysis

As of August 14, 2020, there were a total of 31 subjects in the FAS set. 7 subjects were still under treatment. 1 subject took the drug for more than 12 cycles, 1 subject took the drug for 9-12 cycles, 2 subjects took the drugs for 6-9 cycles, 5 subjects took the drug for 3-6 cycles, 14 subjects took the drug for 1-3 cycles, and 8 subjects took the drug for <1 cycle. Among all subjects, 2 (6.5%) subjects achieved CR, 2 (6.5%) subjects achieved CRh, 4 (12.9%) subjects achieved CRi, and 2 (6.5%) subjects achieved PR. The median EFS of 31 subjects was 3.0 months (95%CI 2.6-5.4) and the median OS thereof was 4.4 months (95%CI 2.9-NC).

Of the 14 subjects in the FAS set of the 150 mg BID group, 1 (7.1%) subject achieved CR at C2, 1 (7.1%) subject achieved CRh at C3, and 2 (14.3%) subjects achieved CRi at C2 and C3 respectively. The CR/CRh cases were 2 (14.3%) in total, and the total CR cases were 4 (28.6%, 95%Cl 8.39-58.1) in total. The median time to the first CR was 2.5 months (in the range of 2-3 months). The median EFS was 4.9 months (95%CI 1.3-5.9). The median OS was 4.2 months (95%CI 2.3-NC). The survival rates for 3-month, 6-month, 9-month, and 11-month were 64.3%, 38.6%, 25.7%, and 25.7%, respectively.

Of the 13 subjects in the FAS set of the 300 mg QD group, 1 subject achieved CR (7.7%) (CRi at C2, CRh at C3, and CR at C4), 1 subject achieved CRh (7.7%) (CRi at C3, and CRh at C4), and 2 subjects achieved CRi (15.4%). The median time to the first CR was 1.5 months (in the range of 1-3 months). The median EFS was 2.9 months (95%CI 1.7-6.2). The median OS was 4.4 months (95%CI 2.7-NC). The survival rates for 3-month and 6-month were 75.2% and 50.1%, respectively.

Of the 4 subjects in the FAS set of the 200 mg BID group, in the efficacy assessment, 3 subjects achieved SD, and 1 subject achieved NE. The median EFS was 2.3 months (95%CI 0.7-3.0). The median OS was 2.9 months (95%CI 0.7-NC). The survival rate for 3-month was 25%.

**Table 11: Overall best efficacy analysis (FAS)**

| | 150mgBID group (N=14) n(%) | 200mgBID group (N=4) n(%) | 300mgQD group (N=13) n(%) | Total (N=31) n(%) |
|---|---|---|---|---|
| CR | 1(7.1) | 0 (0) | 1 (7.7) | 2 (6.5) |
| CRh | 1 (7.1) | 0 (0) | 1 (7.7) | 2 (6.5) |
| CRi | 2 (14.3) | 0 (0) | 2 (15.4) | 4(12.9) |
| PR | 2(14.3) | 0 (0) | 0 (0) | 2 (6.5) |
| SD | 5 (35.7) | 3 (75.0) | 9 (69.2) | 17 (54.8) |
| PD | 1 (7.1) | 0 (0) | 0 (0) | 1 (3.2) |
| NE | 2 (14.3) | 1 (25.0) | 0 (0) | 3 (9.7) |
| Overall CR rate (95%CI) | 28.6 (8.39-58.10) | 0 (0.00-60.24) | 30.8 (9.09-61.43) | 25.8 (11.86-44.61) |

| | | | | |
|---|---|---|---|---|
| Note: Overall CR rate (%)=(CR+CRh+CRi)/enrolled subjects^{∗} 100. | | | | |

For R/R AML, CR, CR duration and CRh were all endpoints that might predict clinical benefit. In addition, the continuous disengagement from blood transfusion was a recognized clinical benefit and provided a support for the CR and CRh maintenance. In this trial, of the 14 subjects in the FAS set of the 150 mg BID dose group, 1 subject achieved CR, 1 subject achieved CRh, and both could disengage from blood transfusion for ≥56 days; of the 13 subjects in the FAS set of the 300 mg QD group, 1 subject achieved CR, 1 subject achieved CRh, and both could disengage from blood transfusion for ≥56 days.

In conclusion, of 27 subjects in the FAS set of 150 mg BID and 300 mg QD groups, 2 subjects achieved CR (7.4%), 2 subjects achieved CRh (7.4%), and the CR/CRh cases were 4 (14.8%) in total. This efficacy result was similar to the efficacy of the 120 mg QD group in a phase 1/2 trial of Gilteritinib in a similar population (Table).

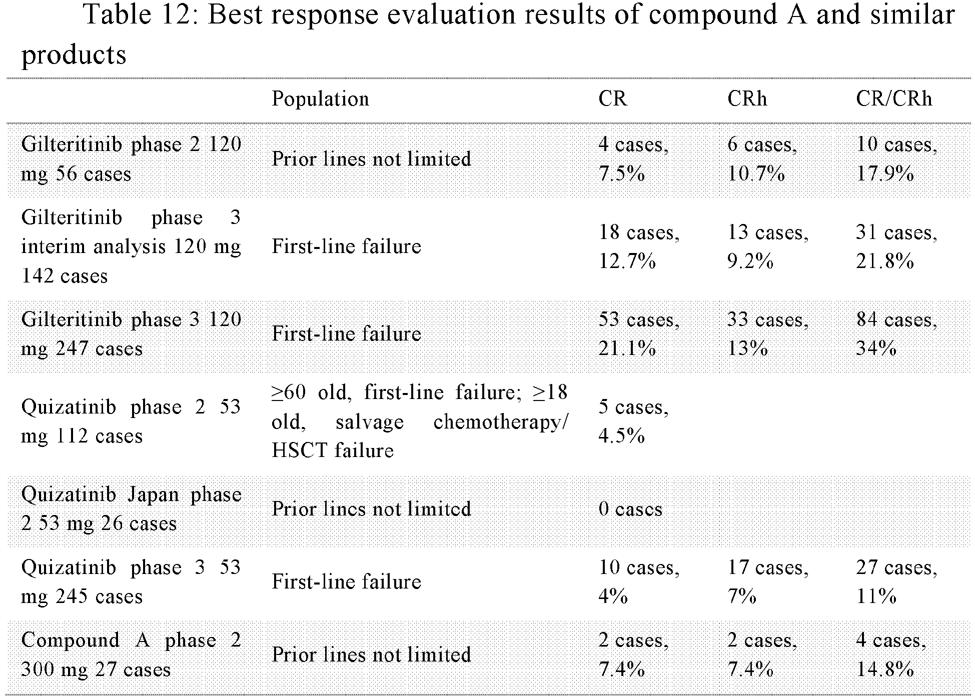

### 2.3.3.2 Influence of unfavorable prognostic factors on efficacy

A multivariate analysis of the prognostic factors of CR had been conducted in a study, and the results identified 6 independent unfavorable factors, including: (1) the duration of the first CR <6 months; (2) the duration of the second CR <6 months; (3) receiving a salvage therapy (excluding allogeneic hematopoietic stem cell transplantation); (4) no chromosome 16 inversion; (5) platelets <50×10⁹/L; (6) leukocytes >50×10⁹/L.

In this trial, the inversion of chromosome 16 of the subjects were not detected, so only the remaining 5 independent unfavorable factors were analyzed, and the results showed:
For 3 subjects with 3 unfavorable factors in the trial, the best responses were all SD, in which 1 subject died in the second cycle, and the other 2 subjects both withdrew the trial in the second cycle.

For 15 subjects with 2 unfavorable factors, 2 subjects achieved CR (unfavorable factors: 1 subject with the durations of CR after both the first and second chemotherapies respectively < 6 months; and 1 subject with the duration of CR after the second chemotherapy < 6 months and receiving a salvage chemotherapy); 1 subject (unfavorable factors: having not ever achieved CR, receiving a salvage chemotherapy, baseline platelets 6 ×10⁹/L) achieved CRi; 1 subject (unfavorable factors: the duration of CR after the first induction <6 months, baseline leukocytes 114.83 ×10⁹/L) achieved PR, and the other subjects achieved SD (in which 1 subject was expected to achieve the remission, the unfavorable factors of this subject: receiving a salvage chemotherapy, and the baseline leukocytes 55.74×10⁹/L). The overall CR rate was 20%.

Of 9 subjects with 1 unfavorable factor, 2 subjects achieved CRi (unfavorable factors: 1 subject with baseline platelets 49× 10⁹/L, 1 subject with receiving a salvage chemotherapy), 1 subject achieved PR, 1 subject was NE, the other subjects all achieved SD. The overall CR rate was 20%.

Of 4 subjects known to have 0 unfavorable factors, 2 subjects achieved CRh, 1 subject achieved CRi, and 1 subject achieved SD. The overall CR rate was 75%.

**Table 13: Unfavorable prognostic factors and efficacy analysis**

| Number of unfavorable prognostic factors(factors) | Number of subjects (subjects) | PD | SD | PR | CRi | CRh | CR | NE | Overall CR rate (%) |
|---|---|---|---|---|---|---|---|---|---|
| 3 | 3 | - | 3 | - | - | - | - | - | 0 |
| 2 | 15 | - | 11 | 1 | 1 | - | 2 | - | 20% |
| 1 | 9 | - | 5 | 1 | 2 | - | - | 1 | 20% |
| 0 | 4 | - | 1 | - | 1 | 2 | - | - | 75% |

To sum up, although the compound A of the present invention had poor efficacy on patients with 3 unfavorable prognostic factors, it had a certain efficacy on subjects with 1-2 unfavorable prognostic factors, and the overall CR rate was 20%. The overall CR rate was as high as 75% in subjects without unfavorable factors.

### 2.3.3.3 Influence of different mutation types on efficacy

Of 31 subjects in this trial, 22 subjects (71.0%) had ITD mutations, and among them, 2 subjects achieved CR, 2 subjects achieved CRh, and 2 subjects achieved CRi; 9 subjects had TKD mutations (29.0%), and among them, 2 subjects achieved CRi in the evaluation of best response. Therefore, the inhibitory effects of compound A on ITD-mutant and TKD-mutant FLT3 were confirmed, and it was expected to be used for the treatment of AML with FLT3-ITD mutation and/or FLT3-TKD mutation.

In this study, the ranked data of ITD mutations of 6 subjects were obtained, and the efficacy was further analyzed. The results showed that: there were 3 subjects with FLT3-ITD^{high} mutation (ITD mutation ≥0.5), and among them, 2 subjects achieved CRh and 1 subject achieved SD (blast cells decreased by 70% at C2 compared with the baseline, and the subject withdrew the trial because the peripheral blood had not been recovered all along); there were 3 subjects with FLT3-ITD^{low} mutation (ITD mutation <0.5), and among them, 1 subject achieved PR as the best response, and 2 subjects achieved SD as the best response. The above limited data suggested that compound A might have better efficacy in AML with FLT3-ITD^{high} mutation.

### 2.3.3.4 Influence of using the prior FLT3 inhibitors on efficacy

Sorafenib, a first-generation type II FLT3 inhibitor, had not yet been approved for treating the indications of relapsed/refractory AML with FLT3 mutation and was currently used off-label. It was recommend in the Chinese guidelines for the diagnosis and treatment of relapsed/refractory AML (2017) and the 2019 NCCN guidelines that patients of relapsed/refractory AML with FLT3-mutation could be treated with sorafenib combined with demethylatingagents.

Type II FLT3 inhibitors bind to inactivated FLT3 receptors and prevent the receptors from being activated. In AML patients, there usually exists the overexpression of FLT3 ligands, especially in the early stage after chemotherapy, and the binding of FLT3 ligands would stimulate the activation of FLT3 receptors, thereby hindering the binding of type II FLT3 inhibitors, leading to drug resistance. In addition, among FLT3 mutations, the TKD mutation is a relatively common point mutation, especially D835. TKD mutations can change the conformation of the point mutation (KD) site, conduce the transition of the FLT3 receptor from an inactive state to an active state, making it difficult for type II FLT3 inhibitors to bind.

In this trial, 10 subjects had received the prior sorafenib treatment, and among them, 1 subject achieved CR, 1 subject achieved CRh, 1 subject achieved CRi, and the other subjects achieved SD. The results suggested that the treatment with compound A could still be beneficial in patients who were refractory to or relapsed with type II FLT3 inhibitors (such as sorafenib).

### 2.3.3.5 Efficacy analysis and discussion of different FAB classifications

Among the subjects enrolled in this trial, there were 6 cases of M2 (19.4%), 9 cases of M4 (29%), and 14 cases of M5 (45.2%) (see Table 10 "FAB classification"). Among the subjects who achieved CR, 1 subject classified as M2, and 1 subject classified as M5; among the subjects who achieved CRh, 1 subject belonged to M4, and 1 subject belonged to M5; among the subjects who achieved CRi, 3 subjects classified as M5, and 1 subject classified as M4. In summary, among the 8 subjects with CR/CRh/CRi, 1 subject classified as M2, 2 subjects classified as M4, and 5 subjects classified as M5. Among subjects who achieved CR/CRh/CRi, the proportion of the M5 subjects was highest (62.5%). The CR/CRh/CRi proportion was analyzed based on the number of enrolled patients with different FAB classfications, the M2 type was 1/6 (16.7%), the M4 type was 2/9 (22.2%), and the M5 type was 5/14 (35.7%). It could be seen that the M5 type proportion is the highest in the achievement of CR/CRh/CRi.

In conclusion, according to the clinical study data collected so far, 150 mg BID and 300 mg QD of compound A showed certain clinical benefits in the treatment of R/R AML with FLT3-mutation. The preliminary efficacy observation results of Compound A were comparable to those of similar products, Quizatinib and Gilteritinib. Compound A still showed good efficacy for AML patients who had failed the treatment with type II FLT3 inhibitors, and AML patients with FLT3-ITD mutation and/or FLT3-TKD mutation. At present, this study was still ongoing, and it was believed that more clinical benefit data would be obtained with the prolongation of the duration of administration and the increase in the number of patients enrolled.

### 2.3.4 Safety assessment

### 2.3.4.1 Adverse events and adverse reactions

As of July 24, 2020, among the 31 subjects enrolled, adverse events occurred before the treatment in a total of 15 subjects (48.4%), among which a serious adverse event occurred in 1 subject.

Adverse events occurred after the treatment in all 31 subjects. Adverse reactions occurred after the treatment in 28 (90.3%) subjects, and the common adverse reactions of grade ≥3 successively included leukocytopenia (16 cases, 51.6%), neutropenia (11 cases, 35.5%), thrombocytopenia (8 cases, 25.8%), anemia (6 cases, 19.4%), Lymphocytopenia (6 cases, 19.4%), diarrhea (5 cases, 16.1%), infectious pneumonia (4 cases, 12.9%) and the like. Throughout the trial, no liver-, kidney-, and heart-related adverse reactions of grade ≥3 occurred.

**Table 14 Summary of adverse events**

| | 150mg group (N=14), n(%) | 200mg group (N=4), n(%) | 300mg group (N=14), n(%) | Total (N=31), n(%) |
|---|---|---|---|---|
| Adverse events | 14 (100.0) | 4 (100.0) | 13 (100.0) | 31 (100.0) |
| Adverse reactions | 12 (85.7) | 4 (100.0) | 12 (92.3) | 28 (90.3) |
| Serious adverse events | 11 (78.6) | 3 (75.0) | 10 (76.9) | 24 (77.4) |
| Serious adverse reactions | 5 (35.7) | 2 (50.0) | 6 (46.2) | 13 (41.9) |
| Adverse events leading to withdrawal from the trial | 3 (21.4) | 1 (25.0) | 0 (0) | 4 (12.9) |
| Adverse reactions leading to withdrawal from the trial | 0 (0) | 0 (0) | 0 (0) | 0 (0) |
| Adverse events leading to dose reduction | 3 (21.4) | 2 (50.0) | 2 (15.4) | 7 (22.6) |
| Adverse reactions leading to dose reduction | 3 (21.4) | 2 (50.0) | 1 (7.7) | 6 (19.4) |
| Adverse events leading to death | 4 (28.6) | 1 (25.0) | 3 (23.1) | 8 (25.8) |
| Adverse reactions leading to death | 1 (7.1) | 0 (0) | 1 (7.7) | 2 (6.5) |
| Important adverse events | 11 (78.6) | 4 (100.0) | 11 (84.6) | 26 (83.9) |
| Important adverse reactions | 8 (57.1) | 3 (75.0) | 8 (61.5) | 19 (61.3) |
| Adverse events of ≥ Grade 3 | 13 (92.9) | 4 (100.0) | 11 (84.6) | 28 (90.3) |
| Adverse reactions of ≥ Grade 3 | 9 (64.3) | 3 (75.0) | 9 (69.2) | 21 (67.7) |
| Non-hematologic adverse events of ≥ Grade 3 | 10 (71.4) | 4 (100.0) | 11 (84.6) | 25 (80.6) |
| Non-hematological adverse reactions of ≥ Grade 3 | 4 (28.6) | 3 (75.0) | 7 (53.8) | 14 (45.2) |
| adverse events of ≥ Grade 4 | 8 (57.1) | 2 (50.0) | 9 (69.2) | 19 (61.3) |
| adverse reactions of ≥ Grade 4 | 7 (50.0) | 2 (50.0) | 9 (69.2) | 18 (58.1) |

### 2.3.4.2 Safety analysis and discussion of each dose group

In the trial, adverse reactions occurred in 12 subjects (85.7%) of 150 mg BID group, 4 subjects (100%) of 200 mg BID group, and 12 subjects (92.3%) of 300 mg QD group. The incidences of adverse reactions, serious adverse reactions, important adverse reactions, adverse reactions of grade ≥3, non-hematological adverse reactions of grade ≥3 and the like in 150 mg BID group were less than those in the other two groups. One adverse reaction leading to death occurred in each of 150 mg BID and 300 mg QD groups. The incidences of hematological adverse reactions of grade ≥4 in 150 mg BID group (7 cases, 50%) and 200 mg BID group (2 cases, 50%) were identical, and both were lower than that in 300 mg QD group (9 cases, 69.2%).

In the vast majority of adverse reactions, the incidence in 150 mg BID group was lower than those in the other two groups. The incidence in 150 mg BID group was lower than those in the other two groups in the vast majority of adverse reactions of grade ≥3, including leukocytopenia, neutropenia, lymphocytopenia, infectious pneumonia, anemia, and the like.

It could be seen that the overall incidence of adverse reactions in 150 mg BID group was lower than those in the other two groups, and 150 mg BID group was advantageous in relatively better safety.

### 2.3.4.3 Comparison in safety with similar products

### (1) Heart-related adverse reactions

In the phase 3 clinical trial of a similar product Quizatinib, the incidence of the serious adverse reaction, QT interval prolongation, was 26.3%; in the phase 3 clinical trial of Gilteritinib, the incidence of QT interval prolongation of all grades was 8.8%, and the incidence of QT interval prolongation of grade ≥3 was 2.5%.

Heart-related adverse reactions such as myocardial cell necrosis and fibrosis, increased heart rate, and QT interval prolongation in dying animals had ever been observed in the preclinical animal experiments of compound A. Therefore, in the clinical trial of compound A, the baseline cardiac conditions of the subjects were strictly controlled, and the heart-related toxicity was closely monitored in the trial.

Among the adverse reactions that occurred in this trial, the adverse reactions that classified as the cardiac organ diseases in accordance with the SOC occurred in a total of 3 subjects (9.7%), of which 2 subjects (14.3%), 0 subjects, and 1 subject (7.7%) were in 150 mg BID group, 200 mg BID group, and 300 mg QD group respectively. Examination abnormality related to the heart in accordance with the SOC occurred in 3 subjects, including elevated blood lactate dehydrogenase (1 subject in 150 mg BID group) and electrocardiogram high voltage (each 1 subject in 200 mg BID group and 300 mg QD group). No adverse reactions of QT interval prolongation were observed in this study. Heart-related adverse reactions that occurred in this trial were all in Grades 1-2,including elevated blood lactate dehydrogenase, heart dilatation, right bundle branch block, peripheral edema, sinus bradycardia, and electrocardiogram high voltage.

### (2) Liver-related adverse reactions

In the phase 3 clinical trial of a similar product Gilteritinib, the incidence of ALT elevation of all grades was 26.3%; the incidence of grade ≥3 was 12.9%; the incidence of AST elevation of all grades was 80.6%, and the incidence of grade ≥3 was 10.3%.

The adverse reactions such as hepatocyte degeneration and necrosis and elevated transaminase had ever been observed in the preclinical animal experiments of compound A. DLT, elevated ALT/AST (related to the concomitant use of estrogen) had ever occurred in 1 subject in the phase I clinical trial of compound A.

Among the adverse reactions that occurred in this trial, the adverse reactions that classified as the hepatobiliary system disease in accordance with the SOC occurred in a total of 4 subjects (12.9%), of which each 2 subjects were in 150 mg BID group and 300 mg QD group (the incidences were 14.3% and 15.4% respectively). Examination abnormality related to the liver and gall in accordance with the SOC included (1) elevated hemobilirubin (2 subjects [14.3%] in 150 mg BID group, 1 subject [25%] in 200 mg BID group, and 3 subjects in 300 mg QD group [23.1%]), a total of 6 subjects [19.4%]); (2) elevated blood alkaline phosphatase (3 subjects [23.1%] in 300 mg QD group, a total of 3 subjects [9.7%]); (3) elevated gamma-glutamyltransferase (2 subjects [15.4%] in 300 mg QD group, a total of 2 subjects [6.5%]); and (4) elevated aspartate amino transferase (1 subject [7.1%] in 150 mg BID group, a total of 1 subject [3.2%]). Hepato toxicities observed in this trial were all in Grades 1-2.

### (3) Serious hematological adverse reactions

In the phase 3 clinical trial of Quizatinib, serious hematological adverse reactions included: thrombocytopenia (34.2%), neutropenia (25.9%), anemia (25.9%), leukopenia (18.0%), febrile neutropenia (15.8%), lymphopenia (4.0%), and cytopenia (2.5%).

Serious hematological adverse reactions caused by compound A in this trial included thrombocytopenia (3 cases, 9.7%), neutropenia (2 cases, 6.5%), leukocytopenia (1 case, 3.2%), bone marrow failure (1 case, 3.2%), anemia (1 case, 3.2%), and hemolyticanemia (1 case, 3.2%).

According to the above results, it could be preliminarily determined that the safety of the medicine of the present invention in the heart, the liver and the blood system was better than that of similar products Quizatinib and Gilteritinib. At present, the number of subjects in this study was relatively low, and further observation was still ongoing.

### 2.4 Overall conclusion

The safety of compound A in the treatment of R/R AML with FLT3-mutation was tolerable in the dosage of 150 mg BID and 300 mg QD, and compound A showed a certain clinical benefit in both 150 mg BID and 300 mg QD groups. The preliminary results showed that compound A was comparable to Quizatinib and Gilteritinib in the efficacy, and had a significantly less incidence of serious cardiac, hepatic and hematological adverse reactions. Compound A still showed good efficacy for AML patients who had failed in the treatment with type II FLT3 inhibitors, AML patients with FLT3-ITD mutation and/or FLT3-TKD mutation, and DEK-CAN positive AML with FLT3-ITD mutation. It is suggested that compound A is expected to treat R/R AML with FLT3 mutation safely and effectively.

### 3. Typical cases

### Case 1:

This subject, female, 21 years old, was admitted to the Department of Hematology, West China Hospital of Sichuan University for treatment on July 14, 2018 due to "diagnosis of AML-M2 for 5+ months". The gene test showed FLT3-ITD mutation, DEK-CAN positive, t (6;9) (p23;q34). The prior use of chemotherapy drugs, such as DA regimen, cytarabine, FLAG, sorafenib+D-HAG, sorafenib+decitabine +HA, did not respond. The percentage of bone marrow myeloblast in the screening period was 78%. Blood routine examination: hemoglobin: 69g/L, platelets: 116^{∗}10⁹/L, neutrophil count: NA.

The single administration stage in the trial of the subject started at a dose of 250 mg QD on July 15, 2018. The multiple administration of the first cycle started on July 18, 2018. After C1, the bone marrow detection showed that the myeloblast was 2.5%, blood routine: hemoglobin: 99g/L, platelets: 135^{∗}10⁹/L, neutrophil count: NA. PR was achieved. The investigator judged that the treatment could be continued. After C2, the bone marrow detection showed that the myeloblast was 0.5%, blood routine: hemoglobin: 134g/L, platelets: 204^{∗}10⁹/L, neutrophil count: 3.09^{∗}10⁹/L, and CR was achieved. As of October 12, 2019, the subject had completed C16. After each cycle of the treatment, the bone marrow detection showed that the myeloblast was ≤2.5%, and CR was continued (except for C1). The latest bone marrow detection (October 12, 2019) showed that the myeloblast percentage was 0 percent.

### Case 2:

This subject (300 mg QD group), female, 37 years old, was diagnosed with AML-M5 with FLT3-ITD mutation on February 15, 2020, and previously received one cycle of HAD regimen (February 15, 2020 to February 21, 2020), one cycle of D-CAG regimen (February 29, 2020 to March 26, 2020), and one cycle of sorafenib regimen (March 06, 2020 to April 2, 2020), the best responses of which were all SDs. This subject was a refractory AML patient. The first administration of compound A was performed on April 14, 2020. After compound A was administered, the subject's myeloblast continued to decline, and CRi was achieved at C3, and the peripheral blood at C4 was recovered compared with the previous cycle, and CRh was achieved. The fourth cycle of administration was completed on August 14, 2020, no dose reduction occurred, and the administration was currently being continued.

### Case 3:

This subject (300mg QD group), female, 23 years old, diagnosed with AML-M5 with FLT3-ITD mutation for 5 years, was a relapsed AML subject. The subject received two cycles of IDA from April 9, 2015 to May 11, 2015, with the best response of CR; one cycle of HD-Arc-c from June 19, 2015 to June 23, 2015, with the best response of CR; one cycle of MEC from December 16, 2019 to December 20, 2019, with the best response of CR; and one cycle of azacitidine from February 21, 2020 to February 27, 2020 with disease progression.

### The subject signed the ICF on April 16, 2020, and the experimental drug was administered for the first time on April 22, 2020, 300 mg QD. The subject had a baseline platelet count of 137 × 10⁹/L, and a leukocyte count of 9.88×10⁹/L.

During the trial, the subject's myeloblast continuously decreased. The myeloblast decreased from 33.5% to 3% in C2, but neutrophils and platelets in hemogram did not reach the CR standard; so the response was assessed as CRi. The myeloblast decreased to 2% in C3 (July 14, 2020), and the peripheral haemocytes partially recovered; CRh was achieved. The administration was currently being continued.

### Case 4:

This subject (150mg BID group), male, 72 years old, was diagnosed with AML-M4 with FL T3-ITD^{high} mutation (unfavourable prognosis) and detected to have NPM1 genes (moderate prognosis). The subject had a disease course of more than 2 months. The subject previously received one cycle of DA regimen (February 14, 2020 to February 18, 2020) and one cycle of IDA regimen (March 8, 2020 to March 14, 2020), the best responses of which were both SDs. This subject was a refractory AML patient.

The first administration was performed on April 23, 2020. The response was significant during the treatment period, reaching CRi at C2 and CRh at C3 (CRh was defined as CRi of platelets ≥ 50×10⁹/L and neutrophils ≥ 0.5×10⁹/L). This subject finished C3 on July 17, 2020, and the administration was currently being continued. No dose reduction occurred to date.

The subject was infused with erythrocytes due to anemia caused by the disease itself in C1, and disengaged from blood transfusion in the subsequent cycles.

### Case 5:

This subject (150mg BID group), female, 32 years old, was diagnosed with AML-M2 with FLT3-ITD mutation and had a disease course of 1.5 years. This subject previously received an IDA chemotherapy regimen and achieved CR as the best response, and she was a relapsed AML patient. The subject achieved CR after 8 cycles of the IDA periodic chemotherapy ending in March 2019. However, the subject relapsed in June 2019. The duration of the first CR was less than 6 months. The patient achieved remission again with a high-dose cytarabine chemotherapy. The subject had a baseline platelet count of 58× 10⁹/L, and a leukocyte count of 1.12 × 10⁹/L.

The first administration of compound A was performed on July 31, 2019. The myeloblast was 3% after C1, and CRi was achieved. However, the investigator considered that the relatively low myeloblast number in the patient was caused by myelosuppression, so the response was strictly judged as PR. After C2, the response was assessed as CR, and the CR response lasted for 5 cycles. On C6D28 (January 17, 2020), the dosage was adjusted to 100mg BID due to the hematopoietic failure by whole blood cells (erythrocytes 2.35×10¹²/L, platelets 163×10⁹/L, neutrophils 1.05×10⁹/L). The actual dose in the evening of C6D28 was 100 mg. The subsequent dosages during C7-C9 were 100 mg BID. Bone marrow examination was not performed due to anemia after C7. The response during C8 and C9 was assessed as SD. Due to the poor response, the dosage was adjusted to 150 mg BID on C10D9 to continue the treatment. The C10 response was assessed as SD, and the C11 response was assessed as CRi. The C12 was completed on July 3, 2020, and the response was assessed as CRi.

The subject was infused with 1 bag of platelets and 2U of erythrocytes on August 22, 2019 (C1D23); 2U of erythrocyte suspension on August 23, 2019; and 1 bag of platelets on August 25, 2019. All above of the blood transfusions were due to the disease itself. The subject was disengaged from blood transfusion in each cycle from August 26, 2019 to July 06, 2020.

The full names in English and the names in Chinese for the following abbreviations used in the specification of this application are as follows:

| | | |
|---|---|---|
| AML | Acute myelocytic leukemia | 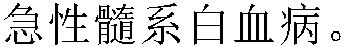 |
| Ara-C | Cytarabine | 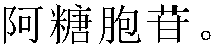 |
| Allo-HSCT | Allogeneic hematopoietic stem cell transplantation | 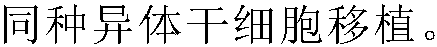 |
| ALT | Alanine aminotransferase | 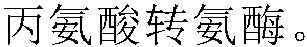 |
| AST | Aspartate aminotransferase | 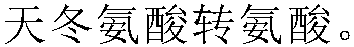 |
| AE | Adverse event | 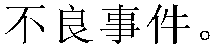 |
| BID | Bis In Die | 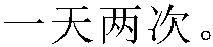 |
| CR | Complete remission | 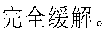 |
| CRi | Complete remission with incomplete hematologic recovery | 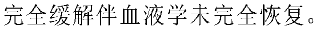 |
| CRh | Complete remission with partial hematologic recovery | 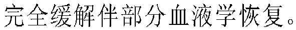 |
| CTCAE | Common Terminology Criteria for Adverse Events | 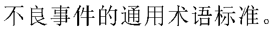 |
| DLT | Dose-Limiting Toxicity | 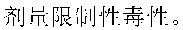 |
| FLT3 | FMS-like tyrosine kinase | FMS 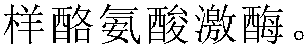 |
| MTD | Maximum tolerated dose | 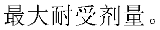 |
| NCI | National Cancer Institute | 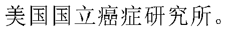 |
| NE | Not be evaluated | 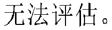 |
| OS | Overall survival | 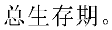 |
| ORR | Objective Response Rate | 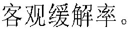 |
| PK | Pharmacokinetic | 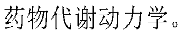 |
| PD | Pharmacodynamic | 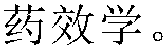 |
| PR | Partial remission | 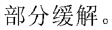 |
| PD | progression disease | 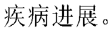 |
| QD | Quaque die | 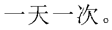 |
| SD | Stable disease | 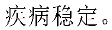 |
| DA | Daunorubicin, cytosinearabinoside | 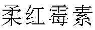 , 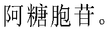 |
| IDA | Idarubicin, cytosinearabinoside | 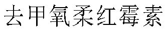 , 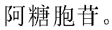 |
| FLAG | Fludarabine, cytosinearabinoside, G-CSF | 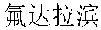 , 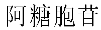 , 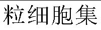 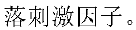 |
| D-HAG | Decitabine, homohrringtonine, cytosinearabinoside, G-CSF | 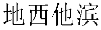 , 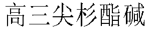 , 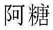 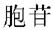 , 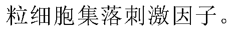 |
| HAD | homohrringtonine, cytosinearabinoside, daunorubicin | 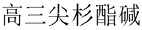 , 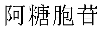 , 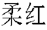 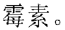 |
| D-CAG | Decitabine, cytosinearabinoside, G-CSF | 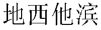 , 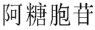 , 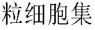 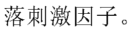 |
| HD-Ara-c | High dose cytosine arabinoside (cytarabine) | 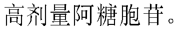 |
| MEC | Mitoxantrone, Etoposide, cytosinearabinoside | 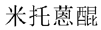 , 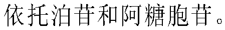 |
| TKD | Tyrosine kinase domain | 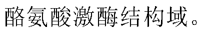 |
| SOC | System Organ Class | 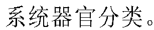 |

## Claims

1. Use of compound A of the following formula or a pharmaceutically acceptable salt thereof in the manufacture of a medicament for the treatment of human leukemia, preferably, the leukemia is acute myeloid leukemia

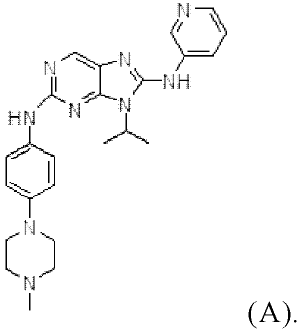

2. Use according to claim 1, wherein the acute myeloid leukemia is selected from relapsed and/or refractory acute myeloid leukemia; or the acute myeloid leukemia is selected from acute myeloid leukemia with FLT3-ITD mutation and/or FLT3-TKD mutation, relapsed and/or refractory acute myeloid leukemia with treatment failure with a Type II FLT3 inhibitor, or DEK-CAN positive acute myeloid leukemia with FLT3-ITD mutation.

3. Use according to claim 2, wherein the Type II FLT3 inhibitor is sorafenib.

4. Use according to claim 1 or 2, wherein the acute myeloid leukemia is acute myeloid leukemia with FL T3-ITD^{high} mutation.

5. Use according to claim 1 or 2, wherein the unfavorable prognostic factors of the acute myeloid leukemia are 0-2.

6. Use according to claim 1 or 2, wherein the FAB classification of the acute myeloid leukemia is subtype M2, M4, or M5, preferably subtype M5.

7. Use according to any of claims 1-6, wherein compound A or a pharmaceutically acceptable salt thereof can be used in the manufacture of the medicament in combination with one or more of other targeted drugs or chemotherapeutic drugs clinically used for the treatment of tumor-related diseases.

8. Use according to any of claims 1-7, wherein the medicament is manufactured into a clinically acceptable formulation, for example oral formulation, injection formulation, topical formulation, and external formulation.

9. Use according to any of claims 1-8, wherein the medicament is a single-dose dosage form or a fractional-dose dosage form; the dosage form contains a therapeutically effective amount of compound A or a pharmaceutically acceptable salt thereof, preferably the therapeutically effective amount is from about 0.001 mg to about 1000 mg, more preferably from about 1 mg to about 500 mg, or from about 20 mg to about 400 mg, or from about 100 mg to about 350 mg, most preferably from about 150 mg to about 330 mg, or from about 160 mg to about 310 mg, or from about 160 mg to about 300 mg; or
the dosage form is a single-dose dosage form of quaquedie (once daily), which contains from about 20 mg to about 500 mg, preferably from about 150 mg to about 400 mg, for example about 150 mg, about 160 mg, about 200 mg, about 250 mg, about 300 mg, about 310 mg, about 350 mg, or about 400 mg of compound A or a pharmaceutically acceptable salt thereof; or
the dosage form is a fractional-dose dosage form of bis in die (twice daily), wherein each fractional dose contains from about 100 mg to about 300 mg, preferably from about 100 mg to about 200 mg, for example about 100 mg, about 125 mg, about 150 mg, about 175 mg, about 200 mg of compound A or a pharmaceutically acceptable salt thereof.

10. A method for treating human acute myeloid leukemia, wherein the method comprises administering to a subject or a patient a therapeutically effective amount of compound A of the following formula or a pharmaceutically acceptable salt thereof, the administering is selected from oral administration, injection administration, local administration, and external administration; the therapeutically effective amount can treat or alleviate the acute myeloid leukemia in the subject or the patient

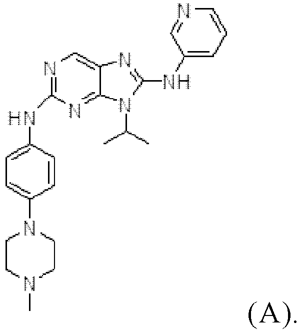

11. The method according to claim 10, wherein the acute myeloid leukemia is selected from relapsed and/or refractory acute myeloid leukemia; or the acute myeloid leukemia is selected from acute myeloid leukemia with FLT3-ITD mutation and/or FLT3-TKD mutation, relapsed and/or refractory acute myeloid leukemia with treatment failure with a Type II FLT3 inhibitor, or DEK-CAN positive acute myeloid leukemia with FLT3-ITD mutation; the Type II FLT3 inhibitor is preferably sorafenib; or
the acute myeloid leukemia is acute myeloid leukemia with FLT3-ITD^{high} mutation; or
the unfavorable prognostic factors of the acute myeloid leukemia are 0-2; or
the FAB classification of the acute myeloid leukemia is subtype M2, M4, or M5, preferably subtype M5.

12. The method according to claim 10 or 11, wherein compound A or a pharmaceutically acceptable salt thereof can be administered in combination with one or more of other targeted drugs or chemotherapeutic drugs clinically used for the treatment of tumor-related diseases.

13. The method according to any of claims 10-12, wherein the therapeutically effective amount is from about 0.001 mg/kg to about 1000 mg/kg; preferably, from about 0.01 mg/kg to about 100 mg/kg omni die (daily); preferably, compound A or a pharmaceutically acceptable salt thereof is administered in a daily dose of from about 0.001 mg to about 1000 mg, preferably from about 1 mg to about 500 mg, or from about 20 mg to about 400 mg, or from about 100 mg to about 350 mg, most preferably from about 150 mg to about 330 mg or from about 160 mg to about 310 mg or from about 160 mg to about 300 mg; adminstered in a single dose or in a fractional dose.

14. The method according to any of claims 10-13, wherein compound A or a pharmaceutically acceptable salt thereof is administered quaque die (once daily), and from about 20 mg to about 500 mg, preferably from about 150 mg to about 400 mg, for example about 150 mg, about 160 mg, about 200 mg, about 250 mg, about 300 mg, about 310 mg, about 350 mg, or about 400 mg is administered each time; or compound A or a pharmaceutically acceptable salt thereof is administered bis in die (twice daily), and from about 100 mg to about 300 mg, preferably from about 100 mg to about 200 mg, for example about 100 mg, about 125 mg, about 150 mg, about 175 mg or about 200 mg is administered each time.

15. Compound A of the following formula or a pharmaceutically acceptable salt thereof for use in the treatment of human acute myeloid leukemia,

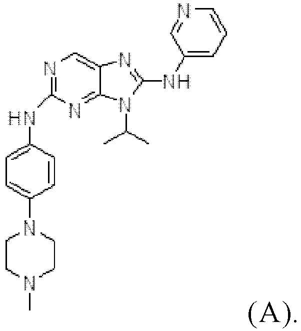

16. The compound A or a pharmaceutically acceptable salt thereof according to claim 15, wherein the acute myeloid leukemia is selected from relapsed and/or refractory acute myeloid leukemia; or the acute myeloid leukemia is selected from acute myeloid leukemia with FLT3-ITD mutation and/or FLT3-TKD mutation, relapsed and/or refractory acute myeloid leukemia with treatment failure with a Type II FLT3 inhibitor, or DEK-CAN positive acute myeloid leukemia with FLT3-ITD mutation; the Type II FLT3 inhibitor is preferably sorafenib; or
the acute myeloid leukemia is acute myeloid leukemia with FLT3-ITD^{high} mutation; or
the unfavorable prognostic factors of the acute myeloid leukemia are 0-2; or
the FAB classification of the acute myeloid leukemia is subtype M2, M4, or M5, preferably subtype M5.

17. The compound A or a pharmaceutically acceptable salt thereof according to claim 15 or 16, wherein compound A or a pharmaceutically acceptable salt thereof can be administered in combination with one or more of other targeted drugs or chemotherapeutic drugs clinically used for the treatment of tumor-related diseases.

18. The compound A or a pharmaceutically acceptable salt thereof according to any of claims 15-17, wherein the treatment comprises administering a therapeutically effective amount of compound A or a pharmaceutically acceptable salt thereof, the therapeutically effective amount is from about 0.001 mg/kg to about 1000 mg/kg; preferably, from about 0.01 mg/kg to about 100 mg/kg omni die (daily); preferably, compound A or a pharmaceutically acceptable salt thereof is administered in a daily dose of from about 0.001 mg to about 1000 mg, preferably from about 1 mg to about 500 mg, or from about 20 mg to about 400 mg, or from about 100 mg to about 350 mg, more preferably from about 150 mg to about 330 mg or from about 160 mg to about 310 mg or from about 160 mg to about 300 mg; administered in a single dose or in a fractional dose.

19. The compound A or a pharmaceutically acceptable salt thereof according to any of claims 15-18, wherein compound A or a pharmaceutically acceptable salt thereof is administered quaque die (once daily), and from about 20 mg to about 500 mg, preferably from about 150 mg to about 400 mg, for example about 150 mg, about 160 mg, about 200 mg, about 250 mg, about 300 mg, about 310 mg, about 350 mg, or about 400 mg is administered each time; or compound A or a pharmaceutically acceptable salt thereof is administered bis in die (twice daily), and about 100 mg to about 300 mg, preferably about 100 mg to about 200 mg, for example about 100 mg, about 125 mg, about 150 mg, about 175 mg or about 200 mg is administered each time.

20. A pharmaceutical composition comprising compound A of the following formula or a pharmaceutically acceptable salt thereof and an optional pharmaceutically acceptable excipient, for use in the treatment of human acute myeloid leukemia,

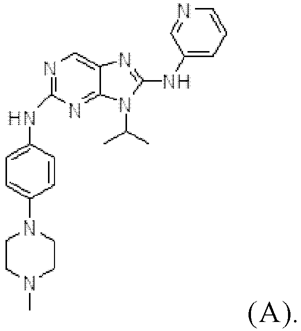

21. The pharmaceutical composition according to claim 20, wherein the acute myeloid leukemia is selected from relapsed and/or refractory acute myeloid leukemia; or the acute myeloid leukemia is selected from acute myeloid leukemia with FLT3-ITD mutation and/or FLT3-TKD mutation, relapsed and/or refractory acute myeloid leukemia with treatment failure with a Type II FLT3 inhibitor, or DEK-CAN positive acute myeloid leukemia with FLT3-ITD mutation; the Type II FLT3 inhibitor is preferably sorafenib; or
the acute myeloid leukemia is acute myeloid leukemia with FLT3-ITD^{high} mutation; or
the unfavorable prognostic factors of the acute myeloid leukemia are 0-2; or
the FAB classification of the acute myeloid leukemia is subtype M2, M4, or M5, preferably subtype M5.

22. The pharmaceutical composition according to claim 20 or 21, wherein the pharmaceutical composition can be administered in combination with one or more of other targeted drugs or chemotherapeutic drugs clinically used for the treatment of tumor-related diseases.

23. The pharmaceutical composition according to any of claims 20-22, wherein the treatment comprises administering a pharmaceutical composition comprising a therapeutically effective amount of compound A or a pharmaceutically acceptable salt thereof, the therapeutically effective amount is from about 0.001 mg/kg to about 1000 mg/kg, preferably, from about 0.01 mg/kg to about 100 mg/kg of compound A or a pharmaceutically acceptable salt thereof omni die (daily); preferably, the pharmaceutical composition is administered in a daily dose of from about 0.001 mg to about 1000 mg, preferably from about 1 mg to about 500 mg, or from about 20 mg to about 400 mg, or from about 100 mg to about 350 mg, more preferably from about 150 mg to about 330 mg or from about 160 mg to about 310 mg or from about 160 mg to about 300 mg of compound A or a pharmaceutically acceptable salt thereof; administered in a single dose or in a fractional dose.

24. The pharmaceutical composition according to any of claims 20-23, wherein the pharmaceutical composition is administered quaque die (once daily) in the dosage of from about 20 mg to about 500 mg, preferably from about 150 mg to about 400 mg, for example about 150 mg, about 160 mg, about 200 mg, about 250 mg, about 300 mg, about 310 mg, about 350 mg, or about 400 mg of compound A or a pharmaceutically acceptable salt thereof each time; or the pharmaceutical composition is administered bis in die (twice daily) in the dosage of from about 100 mg to about 300 mg, preferably from about 100 mg to about 200 mg, for example about 100 mg, about 125 mg, about 150 mg, about 175 mg or about 200 mg of compound A or a pharmaceutically acceptable salt thereof each time.
